# EUROPÄISCHE PATENTSCHRIFT

(11) **EP 2 327 391 B1**
(45) Veröffentlichungstag und Bekanntmachung des Hinweises auf die Patenterteilung: **13.02.2019**
(21) Anmeldenummer: 10187768.6
(22) Anmeldetag: 15.10.2010
(51) Int. Cl.: A61K 8/06, A61Q 17/04, A61K 8/49, A61K 8/02

(54) **Sonnenschutzmittel mit erhöhter Wasserfestigkeit und Verfahren zu dessen Herstellung**
Sunscreen with improved water resistance and method for producing same
Produit de protection solaire doté d'une résistance à l'eau améliorée et son procédé de fabrication

(30) Priorität: 25.11.2009 DE 102009055620
(43) Veröffentlichungstag der Anmeldung: 01.06.2011
(73) Patentinhaber: Beiersdorf AG, 20253 Hamburg (DE)
(72) Erfinder: Skubsch, Kerstin, 25497, Prisdorf (DE); Lerg, Heike, 22303, Hamburg (DE); Köhler, Manuela, 22147 Hamburg (DE)

(56) Entgegenhaltungen:
- EP-A1- 2 351 551
- WO-A2-2010/114710
- US-A1- 2006 275 226
- US-A1- 2008 299 058
- "Polymer dispersions of encapsulated organic UV filters in Personal Care", IP.COM JOURNAL, IP.COM INC., WEST HENRIETTA, NY, US, 12. Januar 2009 (2009-01-12), XP013127766, ISSN: 1533-0001

## Beschreibung

Die vorliegende Erfindung betrifft eine kosmetische Zubereitung mit erhöhter Wasserfestigkeit sowie das Verfahren zu dessen Herstellung.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. So sind in jüngerer Zeit eine Reihe von partikulären organischen Lichtschutzfiltern (beispielsweise Tinosorb M der Firma Ciba) für die Kosmetik entwickelt worden, die zwar in Sonnenschutzmitteln sehr gute UV-Filtereigenschaften zeigen, aber dazu führen, dass die Produkte nur eine geringe Wasserfestigkeit aufweisen. Eine mögliche Ursache für die geringe Wasserfestigkeit könnte darin liegen, dass die partikulären organischen Lichtschutzfilter in wässrigen Dispersionen angeboten werden, die durch oberflächenaktive Alkylglucoside (in der Regel Decyl glucoside) stabilisiert sind. Diese Alkylglucoside dienen darüber hinaus als Hilfsmittel beim Vermahlen der Filterpartikel und sind damit ein fester Bestandteil der kommerziell angebotenen UV-Filterrohstoffe.
Es war daher die Aufgabe der vorliegenden Erfindung, die Wasserfestigkeit (die eine wesentliche Produkteigenschaft für kosmetische Sonnenschutzmittel darstellt) von Zubereitungen mit partikulären organischen Lichtschutzfiltern zu verbessern.
Überraschend gelöst wird die Aufgabe durch ein Verfahren zur Herstellung einer kosmetischen Emulsion enthaltend partikuläre organische Lichtschutzfilter, dadurch gekennzeichnet, dass
a) der in einer wässrigen Zubereitung dispergierte, partikuläre, organische Lichtschutzfilter zusammen mit einem Öl oder einer Mischung aus mehreren Ölen zu einer homogenen Vordispersion dispergiert wird,
b) eine separat vorgemischte, homogene Fettphase mit einer ebenfalls separat vorgemischten wässrigen Phase vereinigt und dispergiert wird und
c) anschließend die Vordispersion a) aus partikulärem Filter und Öl zu diesen vereinigten Phasen unter Rühren hinzugegeben und homogenisiert wird, wobei als partikuläre organische Lichtschutzfilter eine oder mehrere der folgenden Verbindungen eingesetzt werden: Methylene Bis-Benzotriazoyl Tetramethylbutylphenol, 2,4,6-Tris-(biphenyl)-1,3,5-triazin und (2-{4-[2-(4-Diethylamino-2-hydroxy-benzoyl)-benzoyl]-piperazine-1-carbonyl}-phenyl)-(4-diethylamino-2-hydroxy-phenyl)-methanone und nach der Homogenisierung (Verfahrensschritt c)) die Zubereitung auf unter 40 °C abgekühlt, mit temperaturlabilen Inhaltsstoffen, flüchtigen Inhaltsstoffen und/oder Parfümstoffen versetzt und anschließend erneut homogenisiert wird.

Zwar kennt der Fachmann die DE 102004047288 sowie die EP 1606270 und EP 0775698 doch konnten diese Schriften nicht den Weg zur vorliegenden Erfindung weisen. Darüber hinaus kennt der Fachmann die US 2008/299058, WO 2010/114710, US 2006/275226 sowie die Veröffentlichung "Polymer dispersions of encapsulated organic UV filters in Personal Care", IP.com Journal, IP. Com INC, veröffentlicht am 12.. Januar 2009 unter dder IP.com Nummer IPCOM000177948D, die ebenfalls nicht den Weg zur vorliegenden Erfindung weisen konnten.
Das erfindungsgemäße Verfahren ist überraschend einfach, kostengünstig und energiesparend.

Die erfindungsgemäß hergestellten Produkte weisen ein ausgesprochen angenehmes Hautgefühl auf. Sie sind färb-, lager- und temperaturstabil sowie photostabil.

Die nach dem erfindungsgemäßen Verfahren hergestellten Zubereitungen weisen eine wesentlich höhere Wasserfestigkeit auf als herkömmliche Produkte mit partikulären organischen Lichtschutzfiltern, bei denen die wässrige Zubereitung mit den partikulären organischen Lichtschutzfiltern in die Wasserphase eingearbeitet wird (siehe Vergleichsversuch). Die Herstellung der Vordispersion mit Ölen führt im Vergleich zur Herstellung einer Vordispersion mit der gesamten homogenen Fettphase zu deutlich stabileren und homogeneren Produkten.

Die in dieser Beschreibung der Erfindung als mit "erfindungsgemäß", "erfindungsgemäß vorteilhaft" etc. gekennzeichneten Angaben beziehen sich sowohl auf das erfindungsgemäße Verfahren als auch auf das nach dem erfindungsgemäßen Verfahren herstellgestellte Produkt (im Rahmen der Beschreibung Verfahrensprodukt genannt).

Es ist erfindungsgemäß vorteilhaft, wenn die Öle, die mit den partikulären, organischen Lichtschutzfiltern im Schritt a) zur homogenen Vordispersion dispergiert werden, einen Kontaktwinkel aufweisen, der größer ist als 35.

Die Kontaktwinkelmessung erfolgt erfindungsgemäß nach folgender Methode:
Messung des Kontaktwinkels von Lipiden auf Glasobjektträgern zur Bestimmung der Spreitfähigkeit
▪ Messinstrument: Optisches Kontaktwinkelmessgerät Dataphysics OCA 20
▪ Messprinzip: Methode des liegenden Tropfens (sessile drop): Bildanalytische Auswertung des Schattenrisses der Topfenkontur
▪ Tropfenvolumen: 10 µl
▪ Kenngröße: Kontaktwinkel [°] unmittelbar nach Auftropfen
▪ Ausgewertet werden die aufgenommenen Filmsequenzen der Topfen
   ▪ Framerate: 6,25 Bilder/s
   ▪ Aufnahmedauer 3s
▪ Messungen je Probe: 5
▪ Auswertung: Mittelwert der 5 Messwerte
▪ Interpretation: Je kleiner der Winkel, desto größer die Spreitfähigkeit.

**Kontaktwinkel einiger Öle**

| **Öl** | **Kontaktwinkel** |
|---|---|
| Propylheptyl Caprylat | 23° |
| Cyclomethicon | 26° |
| Dicaprylyl Carbonat | 29° |
| Isopropylpalmitat | 38° |
| Butylene Glycol Caprylat/Caprat | 41° |
| Ethylhexyl Salicylat | 41° |
| C12-15 Alkyl Benzoat | 44° |
| Caprylic/Capric Triglyceride | 55° |
| Cocoglycerid | 59° |
| Homomenthylsalicylat | 61° |
| Ethylhexylcinnamat | 65° |
| Mineral Öl | 67° |
| Triisostearin | 75° |
| Octocrylen | 124° |

Das erfindungsgemäße Verfahren und Verfahrensprodukt sind erfindungsgemäß besonders bevorzugt dadurch gekennzeichnet, dass als Öle für die Vordispersion mit dem partikulären organischen Lichtschutzfilter (Schritt a) Mineralöl, Cocoglyceride, Homomenthylsalicylat, Isopropylpalmitat und/oder Triisostearin eingesetzt werden.

Es ist für das erfindungsgemäße Verfahren und Verfahrensprodukt erfindungsgemäß von Vorteil, wenn es sich bei der Emulsion um eine Öl-in-Wasser-Emulsion (O/W-Emulsion) handelt.

In diesem Falle ist es erfindungsgemäß bevorzugt, wenn die Zubereitung einen oder mehrere O/W-Emulgatoren gewählt aus der Gruppe der Verbindungen Glycerylstearatcitrat, Glycerylstearat, Cetearylglucoside, Stearinsäure sowie ihrer Salze, Polyglyceryl-3-methylglycosedistearat, Ceteareth-20, PEG-40 Stearat, PEG-100 Stearat, Natriumstearoyl Glutamat, Natriumcetearylsulfat, Dinatriumcetearylsulfosuccinat enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Das erfindungsgemäße Verfahren und Verfahrensprodukt sind erfindungsgemäß vorteilhaft dadurch gekennzeichnet, dass der Verfahrensschritt c) nach dem Heiß-Heiß-Verfahren durchgeführt wird.

Erfindungsgemäß bevorzugt werden dabei die Produkte der Verfahrensschritte a) und b) vor der Vereinigung auf eine Temperatur von 70 °C bis 90 °C und besonders bevorzugt aul eine Temperatur von etwa 80 °C erhitzt.

Das erfindungsgemäße Verfahren und Verfahrensprodukt sind erfindungsgemäß vorteilhaft dadurch gekennzeichnet, dass die separat vorgemischte, homogene Fettphase (aus Schritt b)) vor der Zugabe der Wasserphase und der Vordispersion, welche die partikulären organischen Lichtschutzfilter enthält, mit den Verdickungsmitteln der Zubereitung versetzt wird.

Erfindungsgemäß bevorzugt werden dabei zunächst die Bestandteile der Fettphase, beispielsweise Wachse, Ester, Fette, Öle aufgeschmolzen. Dies geschieht erfindungsgemäß bei Temperaturen von 70 °C bis 90 °C und besonders bevorzugt bei einer Temperatur von etwa 80 °C. Erfindungsgemäß vorteilhaft ist es also, dass die Zugabe des/der Verdickungsmittel im heißen Zustand der Fettphase erfolgt.

Die Fettphase wird aus den für eine kosmetische Emulsion üblichen lipophilen Bestandteilen zusammengesetzt.

Erfindungsgemäß bevorzugt werden die Verdickungsmittel in die aufgeschmolzene Fettphase eingearbeitet, was beispielsweise dadurch geschehen kann, dass man diese Bestandteile einsinken oder einrühren lässt.

Als Verdickungsmittel können die für eine kosmetische Emulsion üblichen Verdickungsmittel eingesetzt werden, beispielsweise Gummen wie Xanthangummi, Acrylate, Acrylsäure/Vinylpyrrolidon-Crosspolymere, derivatisierte Zellulosen wie Ethylcellulose, Hydroxypropylcellulose, Stärken wie Tapiokastärke und andere Verbindungen.

Als temperaturlabile Inhaltsstoffe kommen beispielsweise Wirkstoffe in Frage wie Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin,

Licochalcon A, Hyaluronsäure, Saponine, Dihydroxyaceton; 8-Hexadecen-1,16-dicarbonsäure, Polydocanol, Parabene, Phenoxyethanol.

Typische Parfümstoff-Bestandteile, die bei diesem Verfahrensschritt zugesetzt werden sind beispielsweise Mischungen aus 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin, Limonen [5989-27-5], Citral, Linalool [78-70-6], alpha-Isomethylionon [1335-46-2], Geraniol [106-24-1] und/oder Citronellol.

Das erfindungsgemäße Verfahren und Verfahrensprodukt sind erfindungsgemäß vorteilhaft dadurch gekennzeichnet, dass zur Herstellung der Vordispersion (Verfahrensschritt a)) die Anteile an wässriger Zubereitung enthaltend dispergierte, partikuläre, organische Lichtschutzfilter zu den Ölen so gewählt wird, dass die Öle mindestens 5 Vol-% dieser Vordispersion ausmachen.

Zum Dispergieren (Herstellen der Vordispersion) bzw. Homogenisieren der verschiedenen Phasen werden übliche Verfahren und Geräte verwendet. Eine Möglichkeit ist z.B. die Verwendung einer Dissolverscheibe und Rührwerk der Firma Pendraulik oder IKA. Auch weitere Rotor-Stator-Homogenisatoren wie z.B. Ultra-Turrax, Homozenta, Becomix oder Krieger oder auch Hochdruckhomogenisatoren können verwendet werden.

Es ist erfindungsgemäß von Vorteil, wenn die Zubereitung in der Fettphase und/oder in der wässrigen Phase weitere UV-Filter enthalten. Dabei ist es erfindungsgemäß bevorzugt, wenn als weitere UV-Filter eine oder mehrere Verbindungen gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat;; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hy-droxy]-phenyl}-6-(4-methoxyphenyl)-1,3,5-triazin (INCI: Bis-Ethylhexyloxyphenol Methoxyphenyl Triazin); 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), 2-Ethylhexyl-2-cyano-3,3-diphenylacrylat (Octocrylen); 4-(tert.-Butyl)-4'-methoxydibenzoylmethan; Titandioxid, Zinkoxid, Merocyanine, Piperazinderivate, eingesetzt werden.

Als erfindungsgemäß vorteilhaftes Piperazinderivat kann dabei die folgende Verbindung eingesetzt werden:

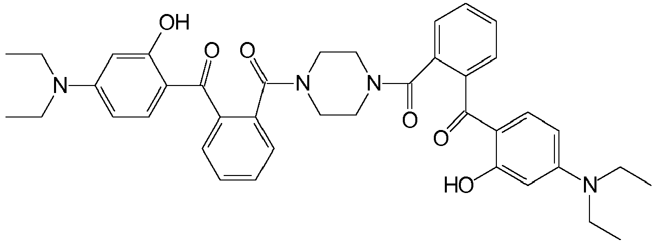

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Es ist erfindungsgemäß von Vorteil, wenn die erfindungsgemäße Zubereitung einen oder mehrere Wirkstoffe enthält, gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1,16-dicarbonsäure, Polydocanol.

Das erfindungsgemäße Verfahren und Verfahrensprodukt sind erfindungsgemäß vorteilhaft dadurch gekennzeichnet, dass die wässrige Zubereitung, in der die partikulären, organischen Lichtschutzfilter dispergiert sind (Verfahrensschritt a)) Alkylglucoside enthält.
Das erfindungsgemäß bevorzugte Alkylglucosid ist dabei jenes mit der INCI Decyl glucoside.

Das erfindungsgemäße Verfahren und Verfahrensprodukt sind erfindungsgemäß vorteilhaft dadurch gekennzeichnet, dass die Partikelgröße der partikulären, organischen Lichtschutzfilter durchschnittlich kleiner/gleich 200 nm beträgt.

Erfindungsgemäß ist nicht zuletzt eine kosmetische Emulsion herstellbar/hergestellt nach einem erfindungsgemäßen Verfahren gemäß der Patentansprüche.

Die erfindungsgemäße Zubereitung kann ein oder mehrere der üblichen Filmbildner zur Erhöhung der Wasserfestigkeit enthalten. Es lassen sich jedoch auch erfindungsgemäß vorteilhafte Zubereitungen ohne dies Filmbildner herstellen.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

### Ausführungsbeispiel/Vergleichsversuch

Das erfindungsgemäße Verfahren und der erfindungsgemäße Effekt konnten beispielhaft mit dem folgenden Versuch gezeigt werden, wobei die Rezeptur und das Herstellungsverfahren 2 erfindungsgemäß ist.

Die in-vivo Wasserfestigkeit wurde mit Hilfe des folgenden Verfahrens bestimmt:
Colipa "Guidelines for Evaluating Sun Product Water Resistence, 2005"

**Rezeptur/Herstellungsverfahren 1**

| | | Bsp.1 |
|---|---|---|
| **Fettphase A** | Ethylhexyl Triazone | 2,5 |
| | VP/Hexadecene Copolymer | 0,5 |
| | Tocopheryl Acetate | 0,1 |
| | Sorbitan Stearate | 0,5 |
| | Stearyl Alcohol | 0,5 |
| | Glyceryl Stearate Citrate | 2 |
| | Bis-Ethylhexyloxyphenol Methoxyphenyl Triazine | 2,5 |
| | Butylene Glycol Dicaprylate/Dicaprate | 9 |
| | Diethylamino Hydroxybenzoyl Hexyl Benzoate | 2,5 |
| | Phenoxyethanol | 0,5 |
| | Hydrogenated Coco-Glycerides | 1 |
| | Myristyl Myristate | 1 |
| | C12-15 Alkyl Benzoate | 9 |
| **Verdickerphase B** | Xanthan Gum | 0,4 |
| | Acrylic Acid/VP Crosspolymer | 0,1 |
| **Wasserphase C** | Methylparaben | 0,2 |
| | Propylparaben | 0,1 |
| | Aqua | 52,05 |
| | Glycerin | 8,6 |
| | 45%-ige Natronlauge | 0,05 |
| | UV-Filter Dispersion (50% 2,4,6-Tris-(biphenyl)-1,3,5-triazin) | 5 |
| | 18%-ige EDTA Lösung | 1 |
| **Parfümphase D** | Parfum | 0,4 |
| | Ethylhexylglycerin | 0,5 |
| | ***in-vivo Wasserfestigkeit,Mittelwert in %*** | ***<10*** |

Die Fettphase A in einer Kitchen-Aid Schüssel auf 80°C im Wasserbad aufschmelzen, bis alles klar gelöst ist. Auf die geschmolzene Fettphase A dann die Verdickerphase B geben und einsinken lassen. Die Wasserphase C in einem Erlenmeyerkolben auf 80°C erwärmen und unter Rühren zu der Fettphase A+B geben. Circa 1 Minute rühren lassen und dann in der Homocenta einmal auf Stufe 1 homogenisieren. In der Kitchen Aid unter Rühren auf ca. 35°C abkühlen lassen. Zugabe der Parfümphase D und noch einmal auf Stufe 1 in der Homocenta homogenisieren.

**Rezeptur/Herstellungsverfahren 2**

| | | Bsp. 2 |
|---|---|---|
| **Fettphase A** | Ethylhexyl Triazone | 2,5 |
| | VP/Hexadecene Copolymer | 0,5 |
| | Tocopheryl Acetate | 0,1 |
| | Sorbitan Stearate | 0,5 |
| | Stearyl Alcohol | 0,5 |
| | Glyceryl Stearate Citrate | 2 |
| | Bis-Ethylhexyloxyphenol Methoxyphenyl Triazine | 2,5 |
| | Butylene Glycol Dicaprylate/Dicaprate | 9 |
| | Diethylamino Hydroxybenzoyl Hexyl Benzoate | 2,5 |
| | Phenoxyethanol | 0,5 |
| | Hydrogenated Coco-Glycerides | 1 |
| | Myristyl Myristate | 1 |
| | C12-15 Alkyl Benzoate | 9 |
| **Verdickerphase B** | Xanthan Gum | 0,4 |
| | Acrylic Acid/VP Crosspolymer | 0,1 |
| **Wasserphase C** | Methylparaben | 0,2 |
| | Propylparaben | 0,1 |
| | Aqua | 52,05 |
| | Glycerin | 8,6 |
| | 45%-ige Natronlauge | 0,05 |
| | 18%-ige EDTA Lösung | 1 |
| **Parfümphase D** | Parfum | 0,4 |
| | Ethylhexylglycerin | 0,5 |
| **UV-Filterphase E** | Cocoglycerides | **3** |
| | UV-Filter Dispersion (50% 2,4,6-Tris-(biphenyl)-1,3,5-triazin) | 5 |
| | ***in-vivo Wasserfestigkeit,Mittelwert in %*** | ***70*** |

Die Fettphase A in einer Kitchen-Aid Schüssel auf 80°C im Wasserbad aufschmelzen, bis alles klar gelöst ist. Auf die geschmolzene Fettphase A dann die Verdickerphase B geben und einsinken lassen.
Für die UV-Filterphase E das Öl Cocoglyceride und UV-Filter Dispersion (50% 2,4,6-Tris-(biphenyl)-1,3,5-triazin) in einem Becherglas einwägen und mit dem Ultraturrax 2 min homogenisieren. (= Vordispergierung des UV-Filter Dispersion (50% 2,4,6-Tris-(biphenyl)-1,3,5-triazin)).
Die Wasserphase C in einem Erlenmeyerkolben auf 80°C erwärmen und unter Rühren zu der Fettphase A+B geben. Zugabe der UV-Filterphase E, anschliessend circa 1 Minute rühren lassen und in der Homocenta einmal auf Stufe 1 homogenisieren. In der Kitchen Aid unter Rühren auf ca. 35°C abkühlen lassen. Zugabe der Parfümphase D und noch einmal auf Stufe 1 in der Homocenta homogenisieren.

**Weitere Beispiele:**

| | | Bsp. 3 | Bsp. 4 | Bsp. 5 | Bsp.6 |
|---|---|---|---|---|---|
| **Fettphase A** | Disodium Cetearyl Sulfosuccinate | 0,5 | 0,5 | 0,5 | |
| | Natrium Stearyl Glutamat | | | | 0,2 |
| | Isopropyl Palmitat | 4 | 4 | 4 | |
| | Octocrylene | 4,5 | 9 | 2 | 5 |
| | Ethylhexylsalicylat | 4,5 | 4,5 | 4,5 | |
| | Butyl Methoxydibenzoylmethan | 4,5 | 4,5 | 2 | 4,5 |
| | VP/Hexadecene Copolymer | 0,5 | 0,5 | 0,5 | 0,5 |
| | Myristyl Myristat | 2 | 2 | 2 | 2 |
| | Phenoxyethanol | 0,5 | 0,5 | 0,5 | 0,5 |
| | Homomenthylsalicylat | | | | 9 |
| **Verdickerphase B** | Xanthan Gum | 0,4 | 0,4 | 0,4 | |
| | Acrylates/C10-30 Alkyl Acrylate | 1 | 1 | 1 | |
| | Acrylic Acid/VP Crosspolymer | | | | 0,8 |
| **Wasserphase C** | Aqua | ad 100 | ad 100 | ad 100 | ad 100 |
| | Glycerin | 1,0 | 5,0 | 3,0 | 4,0 |
| | 45%-ige Natronlauge | q.s. | q.s. | q.s | q.s |
| | 18%-ige EDTA Lösung | 1,0 | 1,0 | 1,0 | 1,0 |
| **Parfümphase D** | Parfum | 0,4 | 0,4 | 0,2 | 0,3 |
| | Tocopheryl Acetate | 0,2 | 0,2 | 0,5 | 0,1 |
| | Ethanol vergällt | 5 | 3 | | 3 |
| | Piroctone Olamine | 0,05 | 0,05 | 0,05 | 0,5 |
| **UV-Filterphase E** | Homomenthylsalicylat | 9 | 9 | 9 | |
| | Isopropyl Palmitat | | | | 3 |
| | UV-Filter Dispersion (50% 2,4,6-Tris-(biphenyl)-1,3,5-triazin) | 6 | 6 | 6 | 4 |
| | UV-Filter Dispersion (50% Methylene Bis-Benzotriazoyl Tetramethylbutylphenol) | | 6 | | |
| | UV-Filter Dispersion (50%(2-{4-[2-(4-Diethylamino-2-HydroxyBenzoyl)-Benzoyl]-Piperazine-1-Carbonyl}-Phenyl)-(4-Diethylamino-2-HydroxyPhenyl)-Methanon) | | | 6 | |

Die Fettphase A bei 80°C aufschmelzen, bis alles klar gelöst ist. Auf die geschmolzene Fettphase A dann die Verdickerphase B geben. Für die UV-Filterphase E das Öl und die UV-Filter Dispersion einwägen und mit dem Ultraturrax 2 min homogenisieren. (= Vordispergierung der UV-Filter Dispersion).
Die Wasserphase C auf 80°C erwärmen und unter Rühren die Phasen A+B dazu geben. Danach unter Rühren Zugabe der UV-Filterphase E, anschliessend circa 1 Minute rühren lassen und homogenisieren. Den Ansatz unter Rühren auf ca. 35°C abkühlen lassen. Zugabe der Parfümphase D und noch einmal homogenisieren.

## Patentansprüche

1. Verfahren zur Herstellung einer kosmetischen Emulsion enthaltend partikuläre organische Lichtschutzfilter, **dadurch gekennzeichnet, dass**
a) der in einer wässrigen Zubereitung dispergierte, partikuläre, organische Lichtschutzfilter zusammen mit einem Öl oder einer Mischung aus mehreren Ölen zu einer homogenen Vordispersion dispergiert wird,
b) eine separat vorgemischte, homogene Fettphase mit einer ebenfalls separat vorgemischten wässrigen Phase vereinigt und dispergiert wird und
c) anschließend die Vordispersion a) aus partikulärem Filter und Öl zu diesen vereinigten Phasen unter Rühren hinzugegeben und homogenisiert wird, wobei als partikuläre organische Lichtschutzfilter eine oder mehrere der folgenden Verbindungen eingesetzt werden: Methylene Bis-Benzotriazoyl Tetramethylbutylphenol, 2,4,6-Tris-(biphenyl)-1,3,5-triazin und (2-{4-[2-(4-Diethylamino-2-hydroxy-benzoyl)-benzoyl]-piparazine-1-carbonyl}-phenyl)-(4-diethylamino-2-hydroxy-phenyl)-methanone und nach der Homogenisierung (Verfahrensschritt c)) die Zubereitung auf unter 40 °C abgekühlt, mit temperaturlabilen Inhaltsstoffen, flüchtigen Inhaltsstoffen und/oder Parfümstoffen versetzt und anschließend erneut homogenisiert wird.

2. Verfahren zur Herstellung einer kosmetischen Emulsion nach Anspruch 1, **dadurch gekennzeichnet, dass** die Öle, die mit den partikulären, organischen Lichtschutzfiltern im Schritt a) zur homogenen Vordispersion dispergiert werden, einen Kontaktwinkel aufweisen, der größer ist als 35.

3. Verfahren zur Herstellung einer kosmetischen Emulsion nach einem der vorhergehenden Ansprüche, **dadurch gekennzeichnet, dass** als Öle für die Vordispersion mit dem partikulären organischen Lichtschutzfilter (Schritt a) Mineralöl, Cocoglyceride, Homomenthylsalicylat, Isopropylpalmitat und/oder Triisostearin eingesetzt werden.

4. Verfahren nach einem der vorhergehenden Ansprüche, **dadurch gekennzeichnet, dass** es sich bei der Emulsion um eine Öl-in-Wasser-Emulsion (O/W-Emulsion) handelt.

5. Verfahren nach einem der vorhergehenden Ansprüche, **dadurch gekennzeichnet, dass** der Verfahrensschritt c)) nach dem Heiß-Heiß-Verfahren durchgeführt wird.

6. Verfahren nach einem der vorhergehenden Ansprüche, **dadurch gekennzeichnet, dass** die separat vorgemischte, homogene Fettphase (aus Schritt b)) vor der Zugabe der Wasserphase und der Vordispersion, welche die partikulären organischen Lichtschutzfilter enthält, mit den Verdickungsmitteln der Zubereitung versetzt wird.

7. Verfahren nach Anspruch 7, **dadurch gekennzeichnet, dass** die Zugabe des/der Verdickungsmittel im heißen Zustand der Fettphase erfolgt.

8. Verfahren nach einem der vorhergehenden Ansprüche, **dadurch gekennzeichnet, dass** zur Herstellung der Vordispersion (Verfahrensschritt a)) die Anteile an wässriger Zubereitung enthaltend dispergierte, partikuläre, organische Lichtschutzfilter zu den Ölen so gewählt wird, dass die Öle mindestens 5 Vol-% dieser Vordispersion ausmachen.

9. Verfahren nach einem der vorhergehenden Ansprüche, **dadurch gekennzeichnet, dass** die Zubereitung in der Fettphase und/oder in der wässrigen Phase weitere UV-Filter enthalten.

10. Verfahren nach einem der vorhergehenden Ansprüche, **dadurch gekennzeichnet, dass** als weitere UV-Filter eine oder mehrere Verbindungen gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 2-(4'-Diethylamino-2'-hydoxybenzoyl)-benzoesäurehexylester; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat;; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), 2-Ethylhexyl-2-cyano-3,3-diphenylacrylat (Octocrylen); 4-(tert.-Butyl)-4'-methoxydibenzoylmethan; Titandioxid, Zinkoxid, Merocyanine, Piperazinderivate, eingesetzt werden.

11. Verfahren nach einem der vorhergehenden Ansprüche, **dadurch gekennzeichnet, dass** die Zubereitung einen oder mehrere Wirkstoffe enthält, gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1,16-dicarbonsäure, Polydocanol.

12. Verfahren nach einem der vorhergehenden Ansprüche, **dadurch gekennzeichnet, dass** die wässrige Zubereitung, in der die partikulären, organischen Lichtschutzfilter dispergiert sind (Verfahrensschritt a)) Alkylglucoside enthält.

13. Verfahren nach einem der vorhergehenden Ansprüche, **dadurch gekennzeichnet, dass** die Partikelgröße der partikulären, organischen Lichtschutzfilter durchschnittlich kleiner/gleich 200 nm beträgt.

## Claims

1. Method for preparing a cosmetic emulsion comprising particulate organic photoprotective filters, **characterized in that**
a) the particulate organic photoprotective filter dispersed in an aqueous preparation is dispersed together with an oil or a mixture of two or more oils to give a homogeneous pre-dispersion,
b) a separately premixed homogeneous fatty phase is combined and dispersed with an aqueous phase that is also separately premixed and
c) subsequently the pre-dispersion a) of particulate filter and oil is added to these combined phases whilst being stirred and is homogenized, wherein the particulate organic photoprotective filters used are one or more of the following compounds: methylene bis-benzotriazoyl tetramethylbutylphenol, 2,4,6-tris(biphenyl)-1,3,5-triazine and (2-{4-[2-(4-diethylamino-2-hydroxybenzoyl)benzoyl]piperazine-1-carbonyl}phenyl)-(4-diethylamino-2-hydroxyphenyl)methanone and, after homogenization (method step c)), the preparation is cooled to below 40°C, temperature-labile ingredients, volatile ingredients and/or perfumes are added, and the mixture is subsequently homogenized again.

2. Method for preparing a cosmetic emulsion according to Claim 1, **characterized in that** the oils which are dispersed with the particulate organic photoprotective filters in step a) to give homogeneous pre-dispersions have a contact angle of greater than 35.

3. Method for preparing a cosmetic emulsion according to either of the preceding claims, **characterized in that** the oils used for the pre-dispersion with the particulate organic photoprotective filter (step a) are mineral oil, cocoglycerides, homomenthyl salicylate, isopropyl palmitate and/or triisostearin.

4. Method according to any of the preceding claims, **characterized in that** the emulsion is an oil-in-water emulsion (O/W emulsion).

5. Method according to any of the preceding claims, **characterized in that** method step c) is carried out by the hot-hot method.

6. Method according to any of the preceding claims, **characterized in that**, to the separately premixed homogeneous fatty phase (of step b)), prior to addition of the water phase and the pre-dispersion, which comprises the particulate photoprotective filters, thickeners of the preparation are added.

7. Method according to Claim 7, **characterized in that** the thickener(s) is/are added to the fatty phase in the hot state.

8. Method according to any of the preceding claims, **characterized in that**, for the preparation of the pre-dispersion (method step a)), the proportions of aqueous preparation comprising dispersed, particulate organic photoprotective filters to the oils is selected such that the oils make up at least 5% by volume of this pre-dispersion.

9. Method according to any of the preceding claims, **characterized in that** the preparation comprises further UV filters in the fatty phase and/or in the aqueous phase.

10. Method according to any of the preceding claims, **characterized in that** the further UV filters used are one or more compounds selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; hexyl 2-(4'-diethylamino-2'-hydroxybenzoyl)benzoate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4-bis(4'-dineopentylaminobenzalmalonate)-6-(4"-butylaminobenzoate)-s-triazine; 4-dicyanomethylene-2,6-dimethyl-1,4-dihydropyridine-N-(ethyloxysulfate ester salt); 2-ethylhexyl 2-cyano-3,3-diphenylacrylate (octocrylene); 4-(tert-butyl)-4'-methoxydibenzoylmethane; titanium dioxide, zinc oxide, merocyanine, piperazine derivatives.

11. Method according to any of the preceding claims, **characterized in that** the preparation comprises one or more active ingredients selected from the group of compounds comprising tocopheryl acetate, tocopherol, glycyrrhetinic acid or salts thereof, alpha-lipoic acid, folic acid, phytoene, D-biotin, coenzyme Q10, alpha-glucosylrutin, carnitine, carnosine, natural and/or synthetic isoflavonoids, glycerylglucose, creatine, creatinine, taurine, β-alanine, licochalcone A, hyaluronic acid, saponins, dihydroxyacetone; 8-hexadecene-1,16-dicarboxylic acid, polidocanol.

12. Method according to any of the preceding claims, **characterized in that** the aqueous preparation, in which the particulate organic photoprotective filters are dispersed (method step a)), comprises alkyl glucosides.

13. Method according to any of the preceding claims, **characterized in that** the average particle size of the particulate organic photoprotective filter is less than/equal to 200 nm.

## Revendications

1. Procédé pour la préparation d'une émulsion cosmétique contenant un filtre particulaire organique de protection contre la lumière, **caractérisé en ce que**
a) le filtre particulaire, organique de protection contre la lumière, dispersé dans une préparation aqueuse, est dispersé conjointement avec une huile ou un mélange de plusieurs huiles en une prédispersion homogène,
b) une phase grasse homogène, prémélangée séparément est rassemblée avec une phase aqueuse également prémélangée séparément et dispersée et
c) la prédispersion a) constituée par le filtre particulaire et l'huile est ajoutée sous agitation à ces phases rassemblées et homogénéisée, un ou plusieurs des composés suivants étant utilisés comme filtre particulaire organique de protection contre la lumière : méthylène-bis-benzotriazoyltétraméthylbutylphénol, 2,4,6-tris-(biphényl)-1,3,5-triazine et (2-{4-[2-(4-diéthylamino-2-hydroxybenzoyl)-benzoyl]-pipérazine-1-carbonyl}-phényl)-(4-diéthylamino-2-hydroxyphényl)-méthanone et après l'homogénéisation (étape de procédé c)), la préparation est refroidie à moins de 40°C, additionnée de constituants labiles à la température, de constituants volatils et/ou de substances parfumées et ensuite de nouveau homogénéisée.

2. Procédé pour la préparation d'une émulsion cosmétique selon la revendication 1, **caractérisé en ce que** les huiles qui sont dispersées avec les filtres particulaires organiques de protection contre la lumière dans l'étape a) en une prédispersion homogène présentent un angle de contact qui est supérieur à 35.

3. Procédé pour la préparation d'une émulsion cosmétique selon l'une quelconque des revendications précédentes, **caractérisé en ce qu'**on utilise, comme huile pour la prédispersion avec le filtre particulaire organique de protection contre la lumière (étape a)), une huile minérale, des glycérides de coco, le salicylate d'homomenthyle, le palmitate d'isopropyle et/ou la triisostéarine.

4. Procédé selon l'une quelconque des revendications précédentes, **caractérisé en ce qu'**il s'agit, pour l'émulsion, d'une émulsion huile-dans-eau (émulsion H/E).

5. Procédé selon l'une quelconque des revendications précédentes, **caractérisé en ce que** l'étape de procédé c) est réalisée selon le procédé chaud-chaud.

6. Procédé selon l'une quelconque des revendications précédentes, **caractérisé en ce que** la phase grasse homogène, prémélangée séparément (de l'étape b)) est additionnée, avant l'addition de la phase aqueuse et de la prédispersion qui contient les filtres particulaires organiques de protection contre la lumière, des épaississants de la préparation.

7. Procédé selon la revendication 7, **caractérisé en ce que** l'addition du ou des épaississants a lieu à l'état chaud de la phase grasse.

8. Procédé selon l'une quelconque des revendications précédentes, **caractérisé en ce que** pour la préparation de la prédispersion (étape de procédé a)), la proportion de préparation aqueuse contenant les filtres particulaires, organiques dispersés de protection contre la lumière par rapport aux huiles est choisie de manière telle que les huiles représentent au moins 5% en volume de cette prédispersion.

9. Procédé selon l'une quelconque des revendications précédentes, **caractérisé en ce que** la préparation contient d'autres filtres UV dans la phase grasse et/ou dans la phase aqueuse.

10. Procédé selon l'une quelconque des revendications précédentes, **caractérisé en ce qu'**un ou plusieurs composés choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels; sels de l'acide 4-(2-oxo-3-bornylidèneméthyl)benzènesulfonique ; sels de l'acide 2-méthyl-5-(2-oxo-3-bornylidèneméthyl)sulfonique ; 2-(2H-benzotriazol-2-yl)-4-méthyl-6-[2-méthyl-3-(1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; ester hexylique de l'acide 2-(4'-diéthylamino-2'-hydroxybenzoyl)-benzoïque ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; salicylate d'homomenthyle ; 2-hydroxybenzoate de 2-éthylhexyle ; diméthicodiéthylbenzalmalonate ; copolymère de 3-(4-(2,2-bis(éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine présentant le n° CAS 285254-16-0 ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine INCI: Ethylhexyl Triazone) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4-bis-(4'-dinéopentylaminobenzalmalonate)-6-(4"-butylaminobenzoate)-s-triazine ; sel d'ester de N-éthoxysulfate de 4-dicyanométhylène-2,6-diméthyl-1,4-dihydropyridine, 2-cyano-3,3-diphénylacrylate de 2-éthylhexyle (Octocrylen) : 4-(tert-butyl)-4'-méthoxydibenzoylméthane ; dioxyde de titane, oxyde de zinc, mérocyanines, dérivés de pipérazine, sont utilisés comme autres filtres UV.

11. Procédé selon l'une quelconque des revendications précédentes, **caractérisé en ce que** la préparation contient une ou plusieurs substances actives choisies dans le groupe des composés acétate de tocophéryle, tocophérol, acide glycyrrhétinique ou ses sels, acide alpha-liponique, acide folique, phytoène, D-biotine, coenzyme Q10, alpha-glucosylrutine, carnitine, carnosine, isoflavonoïdes naturels et/ou synthétiques, glycérylglucose, créatine, créatinine, taurine, β-alanine, licochalcone A, acide hyaluronique, saponines, dihydroxyacétone ; acide 8-hexadécène-1,16-dicarboxylique, polydocanol.

12. Procédé selon l'une quelconque des revendications précédentes, **caractérisé en ce que** la préparation aqueuse dans laquelle les filtres particulaires organiques de protection contre la lumière sont dispersés (étape de procédé a)) contient des alkylglucosides.

13. Procédé selon l'une quelconque des revendications précédentes, **caractérisé en ce que** la grosseur de particule des filtres particulaires organiques de protection contre la lumière est en moyenne inférieure/égale à 200 nm.
